# EUROPEAN PATENT SPECIFICATION

(11) **EP 0 778 946 B1**
(45) Date of publication and mention of the grant of the patent: **23.10.2002**
(21) Application number: 95931030.1
(22) Date of filing: 30.08.1995
(51) Int. Cl.: G01N 33/535, C07D 219/04

(54) **NOVEL METHOD AND KITS FOR PRODUCING LIGHT FROM AN ACRIDAN COMPOUND**
VERFAHREN UND TESTSÄTZE ZUR ERZEUGUNG VON LICHT VON EINER ACRIDAN-VERBINDUNG
NOUVEAU PROCEDE ET KITS POUR PRODUIRE DE LA LUMIERE A PARTIR D'UN COMPOSE ACRIDANE

(30) Priority: 02.09.1994 US 300462
(43) Date of publication of application: 18.06.1997
(73) Proprietor: LUMIGEN, INC., Southfield, Michigan 48034 (US)
(72) Inventor: AKHAVAN-TAFTI, Hashem, MI 48834 (US); ARGHAVANI, Zahra, Brighton, MI 48116 (US); DeSILVA, Renuka, Northville, MI 48167 (US)
(74) Representative: Jones, Helen Marjorie Meredith
(86) International application number: US9511031
(87) International publication number: WO96007912

(56) References cited:
- EP-A- 0 625 510
- US-A- 4 918 192
- US-A- 5 284 951
- JOURNAL OF IMMUNOLOGICAL METHODS, Volume 101, Number 1, issued 16 July 1987, HART et al., "The Use of Acridinium Ester-Labelled Strepavidin in Immunoassays", pages 91-96.

## Description

### BACKGROUND OF THE INVENTION

### (1) FIELD OF THE INVENTION

This invention relates to chemiluminescent acridan compounds particularly N-alkylacridancarboxylic acid derivatives which allow the production of light (chemiluminescence) from the acridan by reaction with a peroxide and a peroxidase. This invention relates to an improved method of generating light chemically (chemiluminescence) by the action of a peroxidase enzyme and an oxidant such as hydrogen peroxide with a group of N-alkylacridancarboxylic acid derivatives. The invention also relates to an improved method of enhancing the amount of chemiluminescence produced from this process by the use of specific enhancer substances. The invention also relates to the use of this method to detect the hydrogen peroxide or peroxidase enzymes. Further, the invention relates to the use of the method to detect and quantitate various biological molecules. For example, the method may be used to detect haptens, antigens, proteins and antibodies by the technique of immunoassay, and DNA or RNA by nucleic acid hybridization assays. The method may additionally be used to detect enzymes which generate hydrogen peroxide such as oxidase enzymes. The method is particularly useful for the detection of biological molecules in assays performed on automated instruments.

### (2) DESCRIPTION OF RELATED ART

The detection and quantitation of biological molecules has been accomplished historically with excellent sensitivity by the use of radiolabeled reporter molecules. Recently numerous non-radioactive methods have been developed to avoid the hazards and inconvenience posed by these materials. Enzyme-linked detection techniques offer the best sensitivity since the catalytic turn over of substrate to produce a detectable change in effect amplifies the detected signal. Substrates which generate color, fluorescence or chemiluminescence have been developed, the latter achieving the best sensitivity.

Further increases in assay sensitivity will expand the range of utility of chemiluminescence-based methods by permitting the detection of analytes present in smaller quantities or reducing the amount of time and/or reagents required to perform the assay. A way to increase the speed and sensitivity of detection in a chemiluminescent assay is to cause the light to be emitted as a short pulse of high intensity.

Horseradish peroxidase (HRP) is one of the most extensively used enzymes in enzyme-linked detection methods such as immunoassays, detection of oligonucleotides and nucleic acid hybridization techniques. Chemiluminescent reagents for HRP detection known in the art do not fully utilize the beneficial properties of this enzyme in analysis mainly due to sensitivity limitations. More efficient chemiluminescent substrates are needed to improve the usefulness of this reporter enzyme. In addition, the ability to generate flashes of high intensity coupled with an enzymatic amplification step, which is unknown in the art, could provide additional benefits in detection sensitivity and automated detection.
a. Oxidation of acridan. Applicants co-pending application, EP-A-0 625 510, and, disclose the use of a peroxidase enzyme to oxidize substituted and unsubstituted N-alkylacridancarboxylic acid derivatives to generate chemiluminescence. In the presence of a peroxidase enzyme and a peroxide, N-alkylacridancarboxylic acid derivatives are efficiently oxidized to produce the N-alkylacridone and blue chemiluminescence in a one-step process.
   Esters of 10-methylacridan-9-carboxylic acid undergo autoxidation to N-methylacridone in dipolar aprotic solvents under strongly basic conditions to produce chemiluminescence (F. McCapra, Acc. Chem. Res., 9(6), 201-8 (1976); F. McCapra, M. Roth, D. Hysert, K.A. Zaklika in *Chemiluminescence and Bioluminescence*, Plenum Press, New York, 1973, pp. 313-321; F. McCapra, *Prog. Org. Chem*., 8, 231-277 (1971); F. McCapra, *Pure Appl. Chem.*, 24, 611-629 (1970); U. S. Patent No. 5,283,334 and 5,284,951 to McCapra and 5,284,952 to Ramakrishnan). Chemiluminescence quantum yields ranged from 10⁻⁵ to 0.1 and were found to increase as the pKₐ of the phenol or alcohol leaving group decreased. Quantum yields in aqueous solution were significantly lower due a competing non-luminescent decomposition of an intermediate. Addition of the cationic surfactant CTAB increased the apparent light yield 130-fold by preventing a competing dark reaction.
b. Chemiluminescent oxidation of acridinium esters. The chemiluminescent oxidation of aliphatic and aromatic esters of N-alkylacridinium carboxylic acid by H₂O₂ in alkaline solution is a well known reaction. The high chemiluminescence quantum yield approaching 0.1 has led to development of derivatives with pendant reactive groups for attachment to biological molecules. Numerous chemiluminescent immunoassays and oligonucleotide probe assays utilizing acridinium ester labels have been reported, for instance J. Immunological Methods 1987, 101, 91-96.
   The use of acridinium esters (AE's), especially when labeled to a protein or oligonucleotide suffers from two disadvantages. The chief problem is limited hydrolytic stability. Acridinium ester conjugates decompose steadily at or slightly above room temperature by hydrolysis of the ester group. Depending on the substitution of the leaving group storage at -20 °C may be required for extended storage. Amides, thioesters and sulfonamides of N-alkylacridinium carboxylic also emit light when oxidized under these conditions (T. Kinkel, H. Lubbers, E. Schmidt, P. Molz, H. J. Skrzipczyk, *J. Biolumin. Chemilumin*., 4, 136-139, (1989), G. Zomer, J. F. C. Stavenuiter, *Anal. Chim. Acta*, 227, 11-19 (1989)). These modified leaving groups only partially improve storage stability.
   A second disadvantage of acridinium esters is the tendency to add nucleophiles such as water at the 9-position to form a non-luminescent pseudo-base intermediate which decomposes in a pH-dependent manner in a dark process. In practice, the pH of solutions containing acridinium esters must be first lowered to reverse pseudo-base formation and then raised in the presence of H₂O₂ to produce light.
   A more fundamental limitation to the use of acridinium esters as chemiluminescent labels lies in the fact that when used as direct labels, only up to at most about 10 molecules can be attached to a protein or oligonucleotide. Coupled with the quantum efficiency for producing a photon (≤ 10%), an acridinium ester-labeled analyte can generate at most one photon of light. In contrast, enzyme-labeled analytes detected by a chemiluminescent reaction can potentially generate several orders of magnitude more light per analyte molecule detected by virtue of the catalytic action of the enzyme.
   An attempt to increase the number of acridinium ester molecules associated with an analyte in an immunoassay was made by constructing an antibody-liposome conjugate wherein the liposome contained an unspecified number of AE's (S.-J. Law, T. Miller, U. Piran, C. Klukas, S. Chang, J. Unger, *J.. Biolumin. Chemilumin.*, 4, 88-98, (1989)). This method only produced a modest increase in signal over a comparable assay using directly labeled AE's.
c. Chemiluminescent Detection of Horseradish Peroxidase. Amino-substituted cyclic acylhydrazides such as luminol and isoluminol react with H₂O₂ and a peroxidase enzyme catalyst (such as horseradish peroxidase, HRP) under basic conditions with emission of light. This reaction has been used as the basis for analytical methods for the detection of H₂O₂ and for the peroxidase enzyme. An analog of luminol (8-amino-5-chloro-7-phenylpyrido[3,4-d]pyridazine-1,4(2H, 3H)dione) has been used in an enhanced chemiluminescent assay with HRP (M. Ii, H. Yoshida, Y. Aramaki, H. Masuya, T. Hada, M. Terada, M. Hatanaka, Y. Ichimori, Biochem. Biophys. Res. Comm., 193(2), 540-5 (1993)). Another chemiluminescent compound oxidized by a peroxidase enzyme and a peroxide is a hydroxy-substituted phthalhydrazide (Akhavan-Tafti co-pending WO-A-9414979. Applicant's co-pending application EP-A-0625510 disclose chemiluminescent N-alkylacridancarboxylic acid esters and sulfonimides which produce light upon reaction with a peroxide and a peroxidase for use in detecting peroxidase enzymes and in assays.

Numerous enhancers have also been employed in conjunction with the use of luminol to increase the intensity and duration of light emitted. These include benzothiazole derivatives such as D-luciferin, various phenolic compounds such as p-iodophenol and p-phenylphenol and aromatic amines (G. Thorpe, L. Kricka, in *Bioluminescence and Chemiluminescence, New Perspectives,* J. Scholmerich, et al, Eds., pp. 199-208 (1987)). For the purposes of the present discussion phenolic compounds are taken to mean hydroxylic aromatic compounds which will also include compounds such as 2-naphthol and 6-bromo-2-naphthol which are known to enhance other peroxidase reactions in addition to the aforementioned substituted hydroxyphenyl compounds. Other compounds which function as enhancers of the chemiluminescent oxidation of amino-substituted cyclic acylhydrazides by a peroxidase are disclosed in U. S. Patent No. 5,206,149 to Oyama and No. 5,171,668 to Sugiyama, PCT application WO 93/16195 dated Aug., 19,1993 and in M. Ii, et al (infra).

### OBJECTS

It is therefore an object of the present invention to provide an improved method and acridan compounds, especially aryl N-alkylacridancarboxylate derivatives with superior properties for use in generating chemiluminescence by the action of a peroxidase enzyme for the detection of biological materials and compounds. It is also an object of the present invention to provide an improved method and kit using aryl N-alkylacridancarboxylate derivatives for use in generating chemiluminescence by the action of a peroxidase enzyme for the detection of peroxidase enzymes and enzyme-conjugates in solution assays. Additionally, it is an object of the present invention to provide an improved method and kit using aryl N-alkylacridancarboxylate derivatives for use in generating chemiluminescence by the action of a peroxidase enzyme for use in nucleic acid assays in solution and on surfaces. Further, it is an object of the present invention to provide an improved method and kit using aryl N-alkylacridancarboxylate derivatives for use in generating chemiluminescence by the action of a peroxidase enzyme for detection of haptens, proteins and antibodies in enzyme immunoassays.

### IN THE DRAWINGS

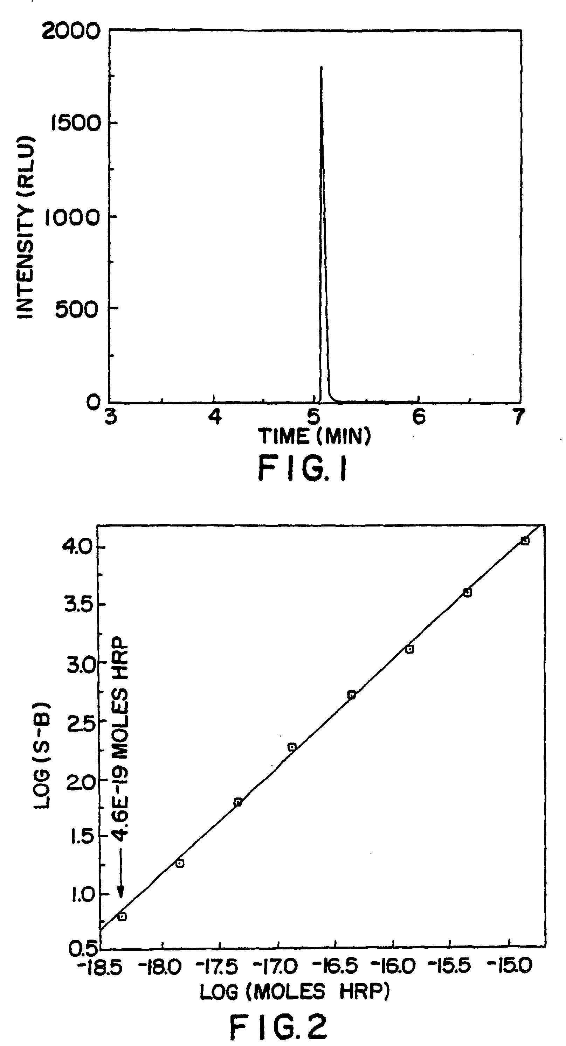

Figure 1 is a graph showing the light emission profile from a reagent containing 2',3',6'-trifluorophenyl 1,6-dimethoxy-10-methylacridan-9-carboxylate (5c) of the present invention. Forty µL of a formulation was incubated with 1 µL of a solution containing 1.4 x 10⁻¹⁶ mol of HRP in water. The formulation consisted of: 1.5 µM acridan compound 5c in 0.01 M tris buffer, pH 8.0, 0.6 mM urea peroxide, 0.1 mM p-phenylphenol, 0.025% TWEEN 20, 1 mM EDTA. After 100 sec, 100 µL of 0.1 M NaOH was injected. The figure shows the intense burst of light emission (in Relative Light Units, RLU) generated under these conditions.

Figure 2 is a graph showing the linearity of detection of HRP using a reagent composition of the present invention. In separate experiments, 50 µL of a solution containing acridan 5c were mixed at room temperature with 1.25 µL aliquots of HRP containing the indicated amounts of enzyme. After 100 sec, 100 µL of 0.1 M NaOH was injected. Light intensity was integrated for 2 sec. The term S-B refers to the chemiluminescence signal (S) in RLU in the presence of HRP corrected for background chemiluminescence (B) in the absence of HRP.

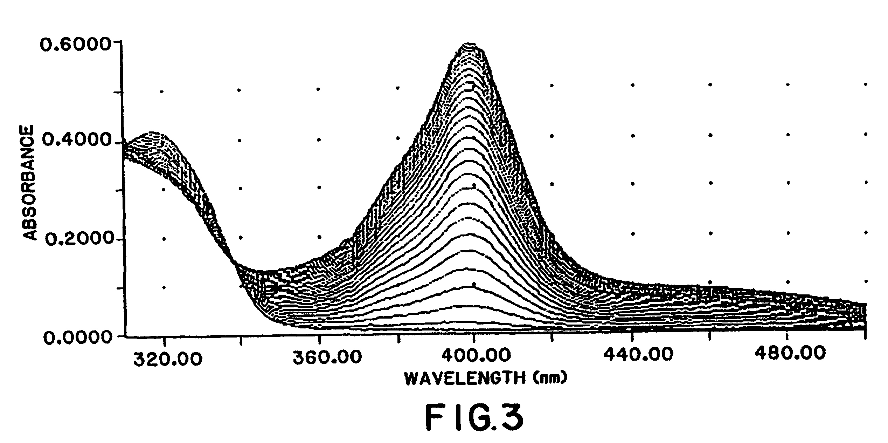

Figure 3 is a graph showing a series of absorption spectra from reaction of the reagent of example 9 containing acridan 5c (3 mL) with 1.1 x 10⁻¹³ mol of HRP. The absorbance spectrum was scanned from 300-500 nm at 30 sec intervals after addition of enzyme. The progression of curves shows the formation (in the direction bottom curve to top curve at 400 nm) of the acridinium compound 4c with an isosbestic point at about 338 nm. After 15 min no further change was observed in the spectrum.

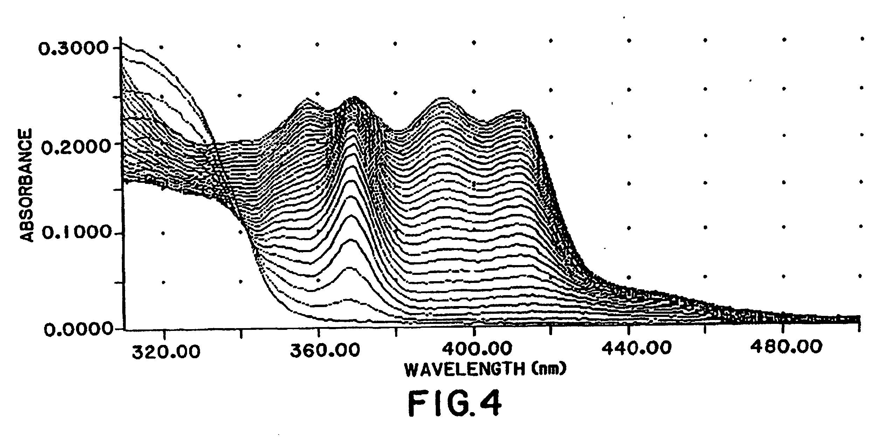

Figure 4 is a graph showing a series of absorption spectra from reaction of 3 mL of a reagent containing the acridan compound 2',6'-difluorophenyl 10-methylacridan-9-carboxylate with 1.1 x 10⁻¹³ mol of HRP. The absorbance spectrum was scanned from 300-500 nm at 30 sec intervals after addition of enzyme. The progression of curves shows a more complex behavior with no isosbestic point indicating the formation (in the direction bottom curve to top curve at 400 nm) of both the acridinium compound 2',6'-difluorophenyl 10-methylacridinium-9-carboxylate and 10-methylacridone as proven by comparison with authentic samples of these two compounds.

The present invention relates to a method for producing chemiluminescence which comprises:
a) reacting a peroxide compound and a peroxidase enzyme with an acridan under conditions of time, temperature and pH at a first level which is in the range 5 to 9 and which permit the accumulation of an intermediate compound wherein the acridan preferably has the formula: 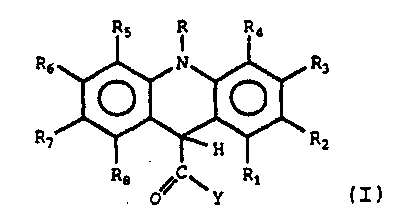 wherein R is selected from alkyl, heteroalkyl and aralkyl groups, wherein R₁ to R₈ are selected independently from groups which allow the production of light and wherein Y is a leaving group; and
b) raising the pH to a level which is at least 10 and high enough to cause the production of a burst of light from reaction of the intermediate with peroxide at an intensity substantially greater than that being produced before raising the pH.

The present invention also relates to the use of this method for detecting an analyte in an assay procedure by a chemiluminescent reaction, wherein the analyte is linked to or capable of being linked directly or indirectly to a peroxidase enzyme and wherein the amount of light produced is related to the amount of the analyte.

The present invention also relates to the use of this method for detecting a peroxidase enzyme in an assay procedure by a chemiluminescent reaction, wherein the amount of light produced is related to the amount of enzyme. The enzyme may be linked to a member of a specific binding pair, for example, by using a biotin-labeled analyte and streptavidin-peroxidase conjugate. Other high affinity binding pairs well known in the art such as fluorescein and anti-fluorescein, digoxigenin and anti-digoxigenin or complementary nucleic acid sequences may also be readily employed as a means of linking a peroxidase enzyme to a member of a specific binding pair for the purpose of practicing this invention. The method may thus be used to detect haptens, antigens, proteins and antibodies by the technique of immunoassay and DNA or RNA by nucleic acid hybridization assays.

The present invention also relates to the use of this method for detecting hydrogen peroxide in an assay procedure by a chemiluminescent reaction with a peroxidase enzyme, wherein the amount of light produced is related to the amount of the peroxide present. It will be apparent to those skilled in the art of chemiluminescent assays that oxidase enzymes may also be detected by use of the present method. Since an oxidase enzyme generates hydrogen peroxide through reduction of oxygen and oxidation of its native substrate, reaction of the hydrogen peroxide produced with an acridan compound of the present invention will produce light which may be related to the amount of the oxidase enzyme.

The present invention also contemplates kits for. detecting any of an analyte, a peroxidase enzyme, a peroxidase enzyme conjugate, a peroxide or a reagent system which produces hydrogen peroxide in an assay procedure by a chemiluminescent reaction. Kits useful for practicing the present invention in any of its embodiments will comprise in one or more containers:
a) an acridan compound as described above;
b) a reagent for raising the pH of the reaction solution to a level of at least 10.
c) a peroxide if the analyte to be detected is not the peroxide or a reagent which generates peroxide;
d) a peroxidase enzyme, if the analyte to be detected *is* not the peroxidase or a conjugate of a peroxidase with the analyte or a conjugate of a peroxidase with a reagent which forms a specific binding pair with the analyte.

In another aspect the present invention relates to a particular acridan compound of the formula:
2',3',6'-trifluorophenyl-1,6-dimethoxy-10-methylacridan-9-carboxylate, and a particular acridinium compound which contains 2',3',6'-trifluorophenyl-1,6-dimethoxy-10-methylacridinium-9-carboxylate.

Preferred groups of compounds used in the invention are: 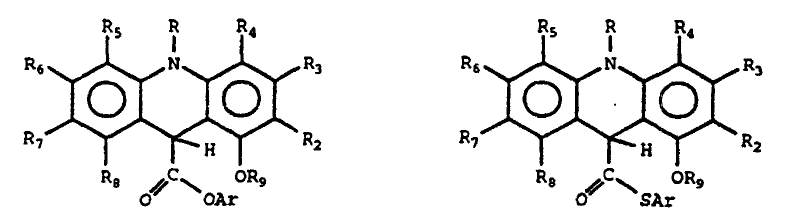 wherein R is an alkyl, aralkyl or heteroalkyl group, wherein R₂₋₈ are selected independently from groups which allow the light to be produced, wherein OR₉ is a C₁ to C₂₀ straight or branched chain alkoxy group and wherein Ar is substituted or unsubstituted aryl or heteroaryl.

Another class of preferred compounds is: 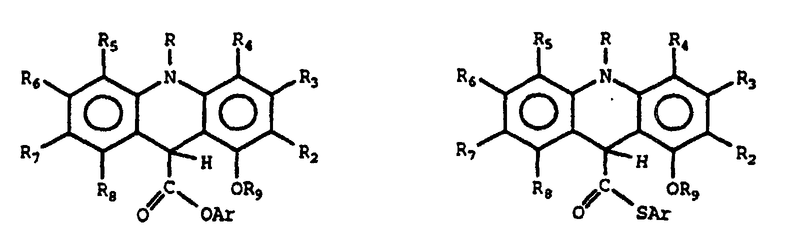 wherein at least one of R₂ through R₈ which may be the same or different are C₁ to C₂₀ straight or branched chain alkoxy groups and wherein OR₉, R and Ar are as defined above.

Another class of preferred compounds is: 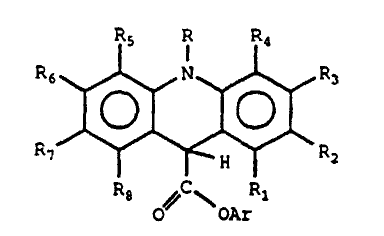 wherein R is an alkyl, aralkyl or heteroalkyl group, wherein R₂₋₈ are selected independently from groups which allow the light to be produced, wherein R₁ is selected from halogens and C₁ to C₂₀ straight or branched chain alkyl groups and wherein Ar is a substituted or unsubstituted aryl or heteroaryl group.

Acridan compounds useful in the practice of the present invention include those with Ar groups consisting of substituted or unsubstituted aryl selected from phenyl, naphthyl, anthryl, phenanthryl and pyrenyl, heteroaryl selected from pyridyl, pyrimidinyl, pyridazinyl, quinolinyl, furyl, benzofuryl, thienyl, imidazolyl and the like. Groups which are contemplated as substituents include alkyl, alkenyl, alkynyl, aralkyl, aryl, alkoxyl, alkoxyalkyl, hydroxyalkyl, halogen, carbonyl, carboxyl, carboxamide, cyano, trifluoromethyl, amino, trialkylammonium and nitro groups.

Substituents may be selected for ease of synthesis or to provide a compound with improved solubility or with particular reaction kinetics. Substituents may also be chosen to provide acridan compounds which have superior stability or diminish side reactions or improve chemiluminescence efficiency as will be appreciated by consideration of the reaction process detailed below and by reference to the examples.

The present invention involves improved acridan compounds which, upon reaction with a peroxidase enzyme and a peroxide compound, are converted into an intermediate compound which subsequently undergoes a rapid chemiluminescent reaction at higher pH. While not wishing to be bound by any particular theory, a plausible identity for such intermediate is the corresponding acridinium compound wherein the center ring is aromatic. Conducting the chemiluminescent reaction in this manner results in a brief burst of light with a high peak intensity. In contrast, light generated by the method disclosed in the EP-A-0 625 510 takes the form of a gradual rise over several minutes to a steady level. Reaction of the acridan with the peroxidase and peroxide will normally be carried out in an aqueous buffer solution at a pH which is compatible with enzyme activity, preferably between about 6 and about 8.5. The pH of the solution is then increased to above about 11 after a preliminary incubation period of a few seconds to several minutes. The intermediate formed by the enzymatic reaction produces a burst of luminescence by reaction with peroxide at the higher pH.

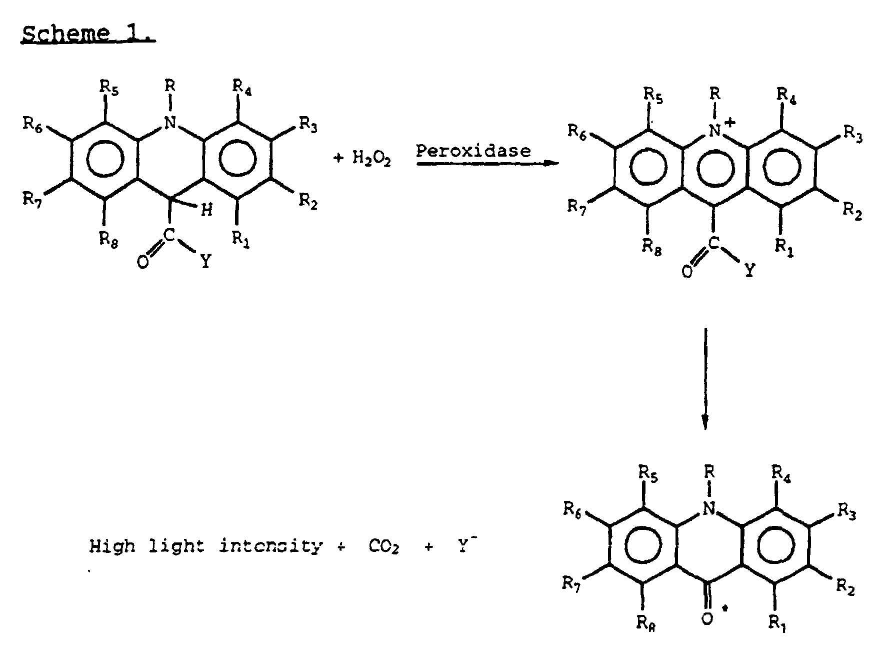

The rate of the chemiluminescent decomposition of the acridinium compound during the enzymatic oxidation phase can be slowed by appropriate choice of acridan compound and by adjusting reaction conditions allowing the acridinium compound to accumulate. For example, acridans with substituents other than hydrogen at the 1-position are oxidized to acridinium compounds with better stability. Subsequently making the reaction solution highly basic greatly accelerates the reaction of the acridinium with peroxide to expel the leaving group and CO₂ and produce light arising from the excited state of the N-substituted acridone.

The chemiluminescent reaction of the present invention provides an unexpectedly sensitive method for detection of peroxidase enzymes or peroxide compounds. The analytical sensitivity as defined by the signal/background ratio is limited by the ability to distinguish the light produced by the base-induced reaction of the enzymatically produced intermediate from all other light producing processes. Quite unexpectedly, three potentially problematic side reactions do not take place to an extent that interferes with the measurement of the desired signal. First, the acridinium ester intermediates formed by enzymatic oxidation of the acridan in the present method produce relatively low levels of light at neutral to moderately alkaline pH. This is surprising in view of the fact that acridinium esters, thioesters and sulfonimides known in the art react rapidly with hydrogen peroxide to produce intense chemiluminescence.

Second, N-alkylacridancarboxylate esters themselves undergo a chemiluminescent reaction (autoxidation) with molecular oxygen at pH ≥ 11 as discussed in McCapra, *infra.* Any unreacted acridan remaining after the incubation with enzyme would therefore be expected to produce a large background emission when the pH was raised. Surprisingly, the acridans of the present invention do not generate a significant quantity of light at the high pH used in the second step relative to the level of light produced from the enzymatically generated acridinium compound.

Third, acridinium compounds can undergo side reactions which do not produce light. Reactions well known in the art which consume the acridinium compound by competing non-luminescent pathways will decrease the amount of light which can be produced. Hydrolysis results in expulsion of the leaving group Y and formation of a non-luminescent carboxylate ion. Addition of nucleophiles to the 9-position results in an intermediate termed a pseudo-base. While this reaction is reversible by lowering the solution pH to about 1 to 3, this would unnecessarily complicate the reaction. Hydrolysis of the starting acridan as well would limit the amount of light which could be produced.

The reaction of the present invention is carried out in solution such as an aqueous buffer which may be in contact with the surface of a solid support such as a bead, tube, membrane or microwell plate coated with enzyme. Suitable buffers include any of the commonly used buffers capable of maintaining a pH in the range of about 6 to about 8.5 for example, phosphate, borate, carbonate, tris(hydroxymethylamino)methane, glycine, tricine, 2-amino-2-methyl-1-propanol, diethanolamine and the like. The preferred method of practicing the invention in this regard is determined by the requirements of the particular intended use.

Incorporation of certain enhancer compounds either alone or in combination with surfactants into the reaction mixture promotes the reactivity of the enzyme. Since the enzymatically produced intermediate undergoes a subsequent chemiluminescent reaction upon raising the pH, the enhanced production of intermediate translates to enhanced production of light. Included among these enhancers are phenolic compounds and aromatic amines known to enhance other peroxidase reactions as described in G. Thorpe, L. Kricka, in *Bioluminescence and Chemiluminescence, New Perspectives*, J. Scholmerich, et al, Eds., pp. 199-208 (1987), M. Ii, H. Yoshida, Y. Aramaki, H. Masuya, T. Hada, M. Terada, M. Hatanaka, Y. Ichimori, *Biochem. Biophys. Res. Comm*., 193(2), 540-5 (1993), and in U.S. Patent Nos. 5,171,668 and 5,206,149 which are incorporated herein by reference. Substituted and unsubstituted arylboronic acid compounds and their ester and anhydride derivatives as disclosed in PCT WO 93/16195, Aug. 19,1993 and incorporated herein by reference are also considered to be within the scope of enhancers useful in the present invention. Preferred enhancers include but are not limited to: p-phenylphenol, p-iodophenol, p-bromophenol, p-hydroxycinnamic acid, 2-naphthol and 6-bromo-2-naphthol.

Additives which suppress the generation of chemiluminescence from the reaction of hydrogen peroxide and aryl acridan derivatives in the absence of peroxidase enzymes are employed to further improve the utility of the invention. It has also been found that certain surfactants such as anionic, cationic and nonionic surfactants improve the sensitivity of detection of the peroxidase enzyme in assays of the present invention by providing a larger signal.

The preferred amounts of the various components of a composition of the present invention are shown in Table I.

**TABLE I**

| | |
|---|---|
| Acridan | 1 nM - 1 mM |
| Phenol enhancer | 1 µM - 10 mM |
| Surfactant | 0.005 - 5 % |
| Peroxide | 0.01 - 10 mM |
| Chelating agent | 0.01 - 5 mM |

The present invention involves a solution in an aqueous buffer containing 1) a phenol enhancer or a salt of a phenol enhancer, 2) a peroxide compound wherein the peroxide compound may be, without limitation, hydrogen peroxide, urea peroxide, or a perborate salt, 3) an acridan compound of the invention, 4) a polydentate cation complexing agent such as EDTA, EGTA and their salts, and 5) a surfactant such as the anionic surfactant sodium dodecyl sulfate (SDS), or preferably a nonionic surfactant such as polyoxyethylenated alkylphenols, polyoxyethylenated alcohols, polyoxyethylenated ethers, polyoxyethylenated sorbitol esters and the like.

In a preferred method of practicing the present invention, an aqueous buffer solution with a pH in the range of about 5 to about 9 containing a phenol compound such as p-phenylphenol or p-iodophenol at a final concentration from about 0.01 M to 1 x 10⁻⁶ M, a nonionic surfactant at a final concentration from about 5 % to 0.005 % (v/v), a peroxide source such as hydrogen peroxide or, preferably, a perborate salt or urea peroxide and a cation complexing agent such as EDTA at a final concentration from about 1 x 10⁻³ M to 1 x 10⁻⁵ M is mixed with a second solution containing an acridan compound of the invention to achieve a final acridan concentration from about 0.001 M to about 1 x 10⁻⁹ M to form the detection reagent solution. This solution is contacted with the peroxidase enzyme which may either be in solution or adhered to a solid support. The detection reaction may be performed over a range of temperatures including at least the range 10 - 40 °C. After an incubation period, the pH of the solution is raised to at least about 10 by addition of a base and, optionally, additional peroxide. As a result, light is produced which rapidly rises to a maximum level and decays. Preferably the addition of base is done rapidly so that the light is emitted over a time interval of a few seconds. Adjustments of incubation time and temperatures and reaction pH as are apparent to the skilled artisan are considered to be within the subject matter of the invention.

A significant advantage of aryl acridan derivatives and compositions of the present invention containing them includes the ability to measure all of the light emitted from the accumulated chemiluminescent product in a short period of time. Measurement of the total light emission which occurs within a period of a few seconds is equivalent to integrating the intensity vs. time curve produced by reaction of applicant's previously disclosed acridans which produce an extended emission. As a result of the time compression of light emission, very small amounts of peroxidase enzyme activity yield large, easily measured spikes of light. This can lead to improved sensitivity of detection if background chemiluminescence is controlled. Assays designed with this type of light detection are readily adapted to existing high volume commercial immunoassay instruments. These and other advantages will be apparent by consideration of the examples.

### EXAMPLES

Synthesis of Acridan Derivatives. Acridancarboxylic acid derivatives were synthesized according to one of the methods shown in Scheme 2 from the corresponding acridine-9-carboxylic acid. 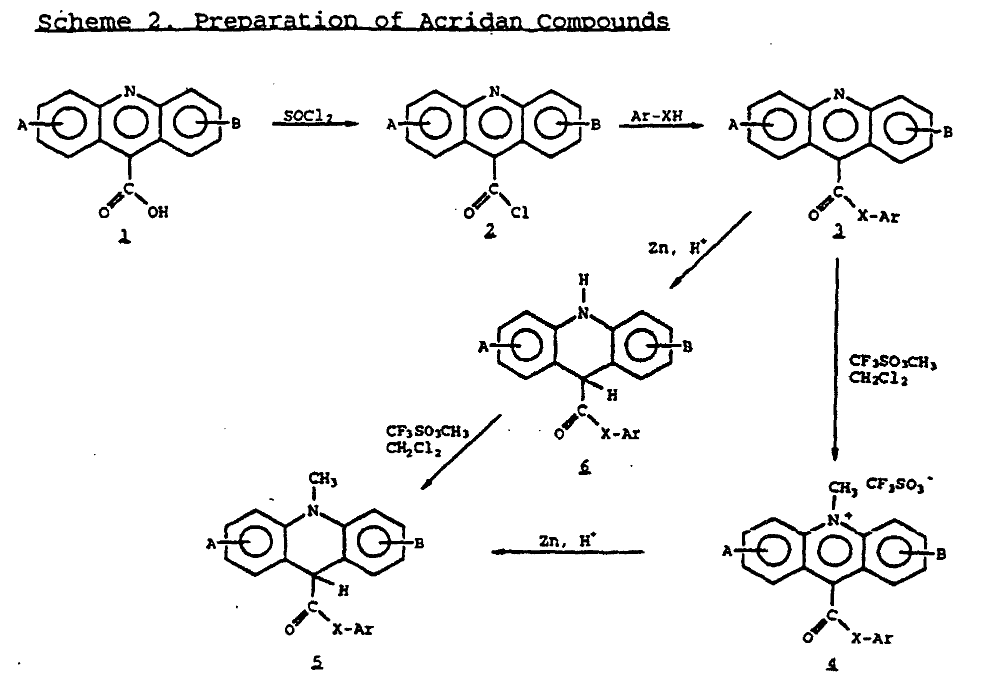 The corresponding acridine-9-carboxylic acid compounds 1a-h were prepared by literature methods (G. Zomer, J. Stavenuiter, R. Van Den Berg, E. Jansen, In Luminescence Techniques in Chemical and Biochemical Analysis, W. Baeyens, D. De Keukeleire, K. Korkidis, eds., Dekker, New York, 505-521, (1991); R. Stollé. *J. Prakt. Chem*., 105, 137, (1922)).

### Example 1. Synthesis of Compound 5a.

(a) condensation of the commercially available (Aldrich) 3-methoxydiphenylamine with oxalyl chloride produced a product (example 1a product).
(b) Example 1a product (1.5 g) was suspended in 10 mL of SOCl₂ and the reaction mixture was refluxed for 3 h. The solvent was removed under reduced pressure to obtain a yellow solid which was dissolved in methylene chloride (CH₂Cl₂) and pyridine (0.7 mL) under argon.
(c) A solution of the phenol (2,3,6-trifluorophenol) (0.878 g) in methylene chloride was added dropwise to the product of example 1b). The solution was stirred overnight at room temperature then diluted with more methylene chloride (100 mL) and washed with water (3 x 50 mL). The organic layer was dried over Na₂SO₄ and concentrated. One compound was isolated by chromatography on silica with 25% ethyl acetate/hexane: ¹H NMR (CDCl₃) δ 4.180 (s), 7.08-8.43 (m).
(d) The compound isolated in example 1c) (0.223 g) was dissolved in 70 mL of ethanol with heating under an atmosphere of argon. After cooling to room temperature and shielding the flask from light, 0.313 g of NH₄Cl (10 eq.) was added followed by 0.382 g (10 eq.) of zinc dust. After 90 min, the solids were filtered off, washed with CH₂Cl₂ and the combined solvents evaporated. The resulting solid material was dissolved in CH₂Cl₂ and filtered yielding a light yellow material. TLC showed the material to be pure and have an R_{f} different from the step a) starting ester.
(e) The reduced product of step d) was methylated with methyl triflate (7 mL) in ca. 8 mL of CH₂Cl₂ under argon with protection from light. After 4 days the volatiles were evaporated and the product chromatographically purified on silica using 50% ethyl acetate/hexane yielding compound 5a as a white solid.

### Example 2. Synthesis of Compound 5b.

(a) A mixture of 10.3 g of Example 1a) product was converted as described in example 1b). The product was dissolved in ca. 200 mL of CH₂Cl₂ under argon. Pyridine (4.2 mL, 1.3 eq.) was added and the solution stirred for 15 min. 4-Fluorothiophenol (5.4 mL, 1.2 eq.) was added and the warm solution stirred over night. Additional CH₂Cl₂ (100 mL) was added and the solution extracted with water and saturated NaCl. The organic layer was dried over Na₂SO₄ and concentrated to a brown solid. The thioester product (3b) was isolated by chromatography on silica with solvents of graded polarity ranging from 20% ethyl acetate/hexane to pure ethyl acetate: ¹H NMR (CDCl₃) δ 4.162 (s), 7.18-7.24 (m), 7.55-7.65 (m), 7.82-7.88 (m), 8.07-8.10 (d), 8.35-8.38 (d).
(b) Thioester 3b (1.19 g) was dissolved in 300 mL of ethanol with heating. After cooling to room temperature and shielding the flask from light, 1.75 g of NH₄Cl (10 eq.) was added followed by 2.14 g (10 eq.) of zinc dust. After 15 min, the solids were filtered off, washed with CH₂Cl₂ and the combined solvents evaporated. The resulting solid material was dissolved in CH₂Cl₂ and filtered yielding a light brown material (6b): ¹H NMR (CDCl₃) δ 3.928 (s), 5.165 (s), 6.56-6.59 (d), 6.89-7.03 (m), 7.16-7.34 (m).
(c) The reduced product 6b with no additional purification was methylated with methyl triflate (12 mL) in 5 mL of CH₂Cl₂ under argon with protection from light. After 72 hours the volatiles were evaporated and the product chromatographically purified on silica using 10-20% ethyl acetate/hexane yielding 0.75 g of white solid 5b): ¹H NMR (CDCl₃) δ 3.712 (s), 3.916 (s), 4.892 (s), 6.656-6.685 (d), 6.97-7.06 (m), 7.12-7.27 (m), 7.31-7.37 (m).

### Example 3. Synthesis of Compound 5c.

(a) A mixture of carboxylic acids (1c, 1d and 1e), (1.4 g, 4.9 mmol) was suspended in excess thionyl chloride (15 mL) and reaction mixture was refluxed for 4 h. The solvent was removed under reduced pressure and the acid chloride product was combined with 2,3,6-trifluorophenol (0.74 g, 5 mmol) and CH₂Cl₂. Pyridine (1 mL, 13 mmol) was added dropwise under argon. The solution was stirred overnight at room temperature and the volatiles removed under reduced pressure. The crude product was subjected to column chromatography on silica using 10 % ethyl acetate/hexane to produce pure 2',3',6'-trifluorophenyl 1,6-dimethoxyacridine-9-carboxylate (3c): ¹H NMR (CDCl₃) δ 4.03 (s, 3H), 4.04 (s, 3H), 6.85-6.88 (d, 1H), 7.02-7.24 (m, 2H), 7.32-7.36 (dd, 1H), 7.48-7.49 (d, 1H), 7.70-7.83 (m, 2H), 8.08-8.11 (d, 1H).
(b) Ester (3c) (0.2 g, 0.46 mmol) was methylated by overnight stirring in 10 mL of CH₂Cl₂ with methyl trifluoromethane-sulfonate (1 mL, 8.8 mmol) under argon. Volatiles were evaporated under reduced pressure and the residue washed with ethyl acetate. N-Methylacridinium ester (4c) was used directly in the next step.
(c) Reduction to the acridan (5c) was accomplished by reacting a solution of 4c (45 mg) and 1 g of NH₄Cl in 25 mL of ethanol with 1 g of zinc. The yellow solution decolorized immediately and was stirred an additional 30 min. Ethyl acetate (50 mL) was added and the mixture filtered. Evaporation of solvents and chromatography of the residue on silica with 10 % ethyl acetate/hexane produced pure 5c: ¹H NMR (CDCl₃) δ 3.39 (s), 3.84 (s, 1H), 3.90 (s, 3H), 5.90 (s, 1H), 6.50-7.43 (m, 8H).

### Example 4. Synthesis of Compound 5d.

(a) Ester 3d was isolated by chromatography from the experiment described in example 3a). ¹H NMR (CDCl₃) δ 4.17 (s), 7.10-7.30 (m), 7.58-7.61 (d), 8.16-8.19 (d).
(b) To a warm solution of ester (0.02 g) and ammonium chloride (2 g) in ethanol (25 ml) was added zinc (2 g) causing immediate decolorization of the solution. The colorless solution was stirred at room temperature for 30 min. Ethyl acetate (200 mL) was added to the solution which was then filtered. The solvents were removed from the filtrate under reduced pressure. The crude material obtained was chromatographed on silica gel (10% ethyl acetate/hexane) to yield the pure product (6d). ¹H NMR (acetone-d₆) δ 3.99 (s), 5.66 (s), 6.87-6.89 (d), 7.12-7.40 (m), 7.43-7.46 (d), 7.58 (s).
(c) The reduced product (0.16g, 0.46 mmol) was methylated with methyl triflate (3 mL) in 5 mL of CH₂Cl₂ under argon with protection from light. After 72 hours the volatiles were evaporated and the product chromatographically purified on silica using 30% ethyl acetate/hexane yielding a white solid: ¹H NMR (CDCl₃) δ 3.47 (s), 3.91 (s), 5.03 (s), 6.74-6.77 (d), 7.80-7.00 (m), 7.21-7.23 (d).

### Example 5. Synthesis of compound 5e. 2',3',6'-Trifluorophenyl 3,6-dimethoxy-10-methylacridan-9-carboxylate.

(a) Ester 3e was isolated by chromatography from the experiment described in example 3a). ¹H NMR (CDCl₃) δ 4.03 (s, 6H), 7.11-7.24 (m, 2H), 7.29-7.33 (dd, 2H), 7.47-7.48 (d, 2H), 8.08-8.11 (d, 2H).
(b) Ester (3e) (1 g, 2.3 mmol) was methylated by overnight stirring in 30 mL of CH₂Cl₂ with methyl triflate (1.3 mL, 5 eq.) under argon. Volatiles were evaporated under reduced pressure and the residue washed with ethyl acetate. N-Methylacridinium ester (4e) was used directly in the next step. ¹H NMR (DMSO-d₆) δ 4.25 (s, 6H), 4.73 (s, 3H), 7.60-8.23 (m, 8H).
(c) Reduction to the acridan (5e) was accomplished by reacting a solution of 4e (650 mg) and 650 mg of NH₄Cl in 100 mL of ethanol with 650 mg of zinc. The yellow solution decolorized immediately and was stirred an additional 30 min. Ethyl acetate (150 mL) was added and the mixture filtered. Evaporation of solvents and chromatography of the residue on silica with 10 % ethyl acetate/hexane produces pure 5e. ¹H NMR (acetone-d₆) δ 3.48 (s, 3H), 3.88 (s, 6H), 5.41 (s. 1H), 6.62-7.39 (m, 8H).

### Example 6. Synthesis of Compound 5f. 2',3',6'-Trifluorophenyl 3-methoxy-10-methylacridan-9-carboxylate.

(a) The product 2',3',6'-trifluorophenyl 3-methoxyacridine-9-carboxylate (3f) was isolated from the reaction of example 1 by chromatography on silica with 25% ethyl acetate/hexane: ¹H NMR (CDCl₃) δ 4.043 (s, 3H), 7.08-8.25 (m, 9H).
(b) Compound 3f (0.24 g) was dissolved in methylene chloride (3 ml) under argon and methyl trifluoromethanesulfonate (0.1 mL, 1.4 eq.) was added. The solution was stirred overnight at room temperature to yield a thick yellow precipitate. This precipitate was filtered, washed with ether and dried to obtain pure 4f as yellow crystals: ¹H NMR (DMSO-d₆) δ 4.288 (s, 3H), 4.837 (s, 3H), 7.64-8.89 (m, 9H).
(d) Compound 4f (35 mg) was suspended in absolute ethanol (15 mL) and solution was refluxed for 10 min to obtain a clear solution. Excess ammonium chloride (4 g) was added by portions to the solution followed by zinc (4 g) causing immediate decolorization of the solution. The colorless solution was refluxed for 30 min, cooled, filtered and the precipitate washed with ethanol (3x20 mL). The solution was concentrated to obtain an off-white solid which was redissolved in methylene chloride and washed with water (3x50 mL). Crude material obtained after evaporation of methylene chloride was chromatographed on silica gel (ethyl acetate/hexane) to yield the pure product as a white solid: ¹H NMR (CDCl₃) δ 3.422 (s, 3H), 3.847 (s, 3H), 5.25 (s, 1H), 6.54-7.39 (m, 9H).

### Example 7. Synthesis of Compound 5g, 2',3',6'-Trifluorophenyl 1,10-dimethylacridan-9-carboxylate.

(a) A mixture of 1-methylacridine-9-carboxylic acid, and 3-methylacridine-9-carboxylic acid (4.0 g, 15.6 mmol) was suspended in excess thionyl chloride (50 mL) and reaction mixture was refluxed for 1.5 h. The solvent was removed under reduced pressure. To the above residue was added 2,3,6-trifluorophenol (2.77 g, 18.7 mmol). This mixture was dissolved in methylene chloride and pyridine (4 ml, 49.5 mmol) was added dropwise under argon. The solution was stirred for several days at room temperature then solvent and excess pyridine was removed under reduced pressure. The crude material obtained was chromatographed on silica gel (5% ethyl acetate/hexane) to yield the isomeric 3 and 1-methylacridine esters: (3g) ¹H NMR (CDCl₃) δ 7.04-7.25 (m, 2H), 7.70-7.77 (m, 3H), 7.87-7.92 (t, 1H), 8.24-8.31 (m, 3H).
(b) The esters 3g (0.2 g) and ammonium chloride (2 g) in ethanol (200 ml) are treated with zinc (2 g) causing immediate decolorization of the solution. The colorless solution is stirred at room temperature for 30 min. Ethyl acetate (200 mL) is added to the solution which is then filtered. The solvents are removed from the filtrate under reduced pressure. The crude material is chromatographed on silica gel (40% ethyl acetate/hexane) to yield 2,3,6-trifluorophenyl 1-methylacridan-9-carboxylate (6g).
(c) The reduced product (0.18g) is methylated with methyl triflate (3 mL) in 5 mL of CH₂Cl₂ under argon with protection from light. After about 72 hours the volatiles are evaporated and the product chromatographically purified on silica using 30% ethyl acetate/hexane to yield (5g) as a white solid.

### Example 8. Synthesis of Compound 5h. 2',3',6'-Trifluorophenyl 1-chloro-10-methylacridan-9-carboxylate.

(a) A mixture of 1-chloroacridine-9-carboxylic acid, and 3-chloroacridine-9-carboxylic acid (4.0 g, 15.6 mmol) was suspended in excess thionyl chloride (50 mL) and reaction mixture was refluxed for 1.5 h. The solvent was removed under reduced pressure. To the above residue was added 2,3,6-trifluorophenol (2.77 g, 18.7 mmol). This mixture was dissolved in methylene chloride and pyridine (4 ml, 49.5 mmol) was added dropwise under argon. The solution was stirred for several days at room temperature then solvent and excess pyridine was removed under reduced pressure. The crude material obtained was chromatographed on silica gel (5% ethyl acetate/hexane) to yield the isomeric 3-chloro- and 1-chloroacridine esters: (3h) ¹H NMR (CDCl₃) δ 7.04-7.25 (m, 2H), 7.70-7.77 (m, 3H), 7.87-7.92 (t, 1H), 8.24-8.31 (m, 3H).
(b) To a warm solution of esters 3h (0.2 g, 0.52 mmol) and ammonium chloride (2 g) in ethanol (200 ml) was added zinc (2 g) causing immediate decolorization of the solution. The colorless solution was stirred at room temperature for 30 min. Ethyl acetate (200 mL) was added to the solution which was then filtered. The solvents were removed from the filtrate under reduced pressure. The crude material obtained was chromatographed on silica gel (40% ethyl acetate/hexane) to yield the pure product 2,3,6-trifluorophenyl 1-chloroacridan-9-carboxylate (6h). ¹H NMR (CDCl₃) δ 5.72 (s), 6.29 (s), 6.68-6.70 (d), 6.77-6.79 (d), 6.79-6.88 (m), 6.96-7.03 (m), 7.12-7.17 (t), 7.21-7.27 (d), 7.54-7.76 (d).
(c) The reduced product 6h (0.18g, 0.46 mmol) was methylated with methyl triflate (3 mL) in 5 mL of CH₂Cl₂ under argon with protection from light. After 72 hours the volatiles were evaporated and the product chromatographically purified on silica using 30% ethyl acetate/hexane yielding a white solid (5h) : ¹H NMR (CDCl₃) δ 3.441 (s, 3H), 5.754 (s, 1H), 6.81-7.09 (m, 6H), 7.22-7.38 (m, 2H), 7.52-7.55 (dd, 1H).

### Chemiluminescence Measurements

The experiments in the following examples were performed using either a Turner Designs TD-20e (Sunnyvale, CA) luminometer fitted with neutral density filter for light attenuation or a Labsystems Luminoskan (Helsinki, Finland) luminometer. Data collection, analysis and display were software controlled.

Example 9. A detection reagent was prepared by combining in a 40:1 ratio reagent A consisting of: 0.6 mM urea peroxide, 0.1 mM p-phenylphenol, 0.025% TWEEN 20, 1 mM EDTA in 0.01 M tris buffer, pH 8.0 and reagent B consisting of: acridan 5c (0.86 mg/mL) in 1:1 (v/v) p-dioxane/ethanol or 1:1 (v/v) propylene glycol/ethanol. To 40 µL of the resulting solution, 1 µL of HRP ((1.4 x 10⁻¹⁶ mol)) was added and the solution incubated for 5 min. A flash of luminescence was induced by injecting 100 µL of 0.1 M NaOH solution. A blank was performed by repeating the experiment without the addition of enzyme. Figure 1 shows the generation of light emission which resulted.

Example 10. An experiment according to example 9 was repeated using a detection reagent prepared by combining reagents A and B in a 1200:1 ratio and an incubation time of 100 sec. A better signal/background ratio resulted due to a lowering of the light intensity of the blank.

Example 11. The sensitivity and linearity of detection of HRP using the detection reagent of example 10 was determined. In each of 3 wells of a microplate, 50 µL volumes of the detection reagent were mixed at room temperature with 1 µL aliquots of solutions of HRP containing between 1.4 x 10⁻¹⁵ and 1.4 x 10⁻¹⁹ mol of enzyme. After 100 sec, 100 µL of 0.1 M NaOH was added and luminescence integrated for 2 sec. Figure 2 shows the linear range of HRP amount measured using a reagent of the present invention containing acridan 5d.

Example 12. A detection reagent according to the composition of example 9 containing instead the acridan 5b was tested for detection of HRP. The method specified in example 10 was followed with the exception that the detection solution was incubated with the enzyme for 5 min. The lowest detected amount of HRP was 1.4 amol (1.4 x 10⁻¹⁸ mol) with a signal/background ratio of 2.

Example 13. A detection reagent according to the composition of example 9 containing instead a slightly impure preparation of the acridan 5d was tested for detection of HRP. Following the method specified in example 10, 1.4 x 10⁻¹⁶ mol of HRP incubated with the reagent and flashed with NaOH produced a signal 105 times greater than the blank. Using 1.4 x 10⁻¹⁷ mol of HRP and incubating for 10 min produced a signal 69 times greater than the blank. The principal impurity, the N-demethylated analog (6d) was tested independently under the conditions of the experiment and found not to produce a significant amount of light.

Example 14. A detection reagent containing instead a crude preparation of the acridan 5h was tested for detection of HRP. Following the method specified in example 10, 1.4 x 10⁻¹⁶ mol of HRP incubated with the reagent for 3 min and treated with 0.1 M NaOH produced a signal 72 times greater than the blank. The principal impurity, the N-demethylated analog (6h) was tested independently under the conditions of the experiment and found not to produce light.

Example 15. Effect of Enhancers. Detection reagent solutions according to the composition of example 9 may be prepared with substitution of various phenolic enhancers, reacted with HRP and subsequently made highly basic. Useful levels of light intensity compared to reagent background are obtained with reagents incorporating p-iodophenol, p-bromophenol, p-hydroxycinnamic acid, 2-naphthol, 6-bromo-2-naphthol and 4-iodophenylboronic acid.

Example 16. Effect of Peroxide. Detection reagent solutions according to the compositions of example 9 may be prepared with substitution of various peroxides, reacted with HRP and subsequently made highly basic. Useful levels of light intensity compared to reagent background are obtained with reagents incorporating hydrogen peroxide, sodium perborate and urea peroxide.

Example 17. A solution of the reagent of example 9 containing acridan 5c (3 mL) was placed in a quartz cuvette in a Varian Cary 3E (Palo Alto, CA) UV-Vis spectrophotometer. HRP (1.4 x 10⁻¹⁵ mol) was added and the absorbance spectrum between 300-500 nm scanned at 30 sec intervals. Figure 3 shows the formation (in the direction bottom curve to top curve at 400 nm) of the acridinium compound 4c. After 15 min no further change was observed in the spectrum. A 0.1 M NaOH solution was added causing a burst of blue light. The spectrum of the resulting solution matched the absorption of 1,6-dimethoxy-10-methylacridone.

Example 18. To demonstrate that acridans bearing substituents in the 1-position provide superior performance, the experiment of example 17 was repeated using an acridan compound which is unsubstituted at the 1-position. A solution of a reagent similar to that used in example 9 (3 mL) but containing the acridan 2',6'-difluorophenyl 10-methylacridan-9-carboxylate was reacted with HRP (1.4 x 10⁻¹⁵ mol) and the absorbance spectrum between 300-500 nm scanned at 30 sec intervals. Figure 4 shows the formation (in the direction bottom curve to top curve at 400 nm) of both the acridinium compound 2',4'-difluorophenyl 10-methylacridinium-9-carboxylate and 10-methylacridone as proven by comparison with authentic samples of these two compounds. In addition, subjecting the acridinium compound 2',4'-difluorophenyl 10-methylacridinium-9-carboxylate to the same reagent formulation in the absence of added HRP led to measurable conversion to the same acridone within minutes.

## Claims

1. A method for producing chemiluminescence comprising:
(a) reacting a peroxide compound and a peroxidase enzyme with an acridan under conditions of time, temperature and pH at a first level which is in the range 5 to 9 and which permit the accumulation of an intermediate compound; and
(b) raising the pH to a second level which is at least 10 and high enough to cause the production of a burst of light from reaction of the intermediate with peroxide at an intensity substantially greater than that being produced before raising the pH.

2. The method of Claim 1 wherein the acridan has the formula: 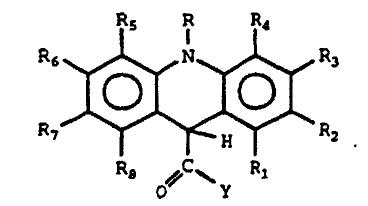 wherein R is selected from alkyl, heteroalkyl and aralkyl groups, wherein R₁ to R₈ are selected independently from groups which allow the production of light and wherein Y is a leaving group.

3. The method of Claim 2 wherein R₁ is a group selected from alkyl, alkoxy and halogen groups.

4. The method of Claim 3 wherein at least two substituents on the acridan ring are selected from alkyl and alkoxy groups.

5. The method of Claim 1 wherein the acridan is of the formula: 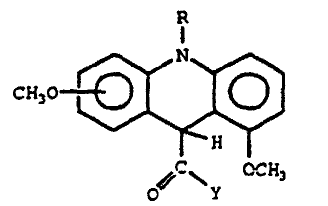 wherein R is selected from alkyl, heteroalkyl and aralkyl groups and wherein Y is a leaving group.

6. The method of Claim 2 wherein the group Y is a phenoxy group substituted with at least one fluorine atom.

7. The method of Claim 1 wherein the acridan is 2',3',6'-trifluorophenyl 1,6-dimethoxy-10-methylacridan-9-carboxylate.

8. the method of claim 1 wherein the pH in step (a) is in the range 6 to 8.5 and the pH is step (b) is above 11.

9. A method according to claim 1 or claim 2 which is for detecting the presence or amount of an analyze in an assay procedure In which the amount of light produced is observed and is related to the presence or amount of the analyte.

10. The method of Claim 9 wherein the analyte is the peroxide.

11. The method of Claim 9 wherein the analyte is the peroxidase enzyme.

12. The method of Claim 8 wherein the peroxidase enzyme is linked to a member of a specific binding pair selected from the group consisting of haptens, antigens, antibodies and oligonucleotides.

13. A kit for detecting the presence or amount of an analyte in an assay procedure by a chemiluminescent reaction which produces an intermediate compound, comprising providing :
(a) an acridan compound;
(b) a peroxide in an aqueous solution at a pH at a first level which is in the range 5 to 9 and which allows formation of an intermediate compound;
(c) a peroxidase enzyme either singly or attached to an analyte-binding compound; and
(d) a reagent for raising the pH of the solution to a second level, wherein light is detected in the assay procedure by reacting the acridan compound with the peroxide and the peroxidase enzyme to form an intermediate compound and subsequently raising the pH of the solution to the second level, which is at least 10, with the reagent.

14. The kit of Claim 13 wherein the acridan has the formula: 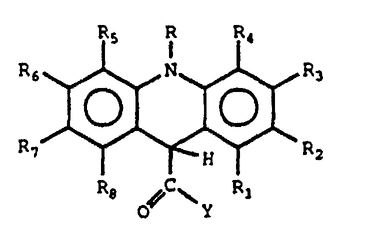 wherein R is selected from alkyl, heteroalkyl and aralkyl groups, wherein R₁ to R₈ are selected independently from groups which allow the production of light and wherein Y is a leaving group.

15. A kit according to claim 14 in which the acridan is in a reagent composition with a phenolic compound which enhances light production from the acridan; a peroxide compound which participates in the reaction of the acridan with the peroxidase; a chelating agent which prevents the peroxide compound from reacting prior to addition of the peroxidase to the composition; and a surfactant in an amount which provides improved chemiluminescence.

16. The kit of Claims 14 or 15 wherein R₁ is a group selected from alkyl, alkoxy and halogen groups.

17. The kit of Claim 16 wherein at least two substituents on the acridan ring are selected from alkyl and alkoxy groups.

18. The kit of Claim 14 or 15 wherein the group Y is a phenoxy group substituted with at least one fluorine atom.

19. The kit of any of Claims 13, 14 or 15 wherein the acridan has the formula: 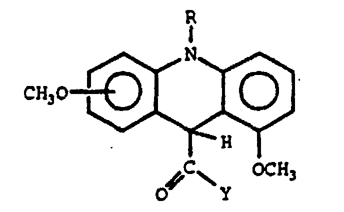 wherein R is selected from alkyl, heteroalkyl and aralkyl groups and wherein Y is a leaving group.

20. The kit of any of Claims 13, 14 or 15 wherein the acridan has the formula 2',3',6'-trifluorophenyl 1,6-dimethoxy-10-methylacridan-9-carboxylate.

21. A compound which is 2',3',6'-trifluorophenyl 1,6-dimethoxy-10-methylacridan-9-carboxylate.

22. A compound which contains 2',3',6'-trifluorophenyl 1,6-dimethoxy-10-methylacridinium-9-carboxylate.

## Patentansprüche

1. Verfahren zur Erzeugung einer Chemilumineszenz, das Folgendes umfasst:
(a) Umsetzen einer Peroxidverbindung und eines Peroxidaseenzyms mit einem Acridan unter Zeit-, Temperatur- und pH-Wert-Bedingungen - pH-Wert auf einer ersten Stufe im Bereich zwischen 5 und 9 - die die Anreicherung einer Zwischenverbindung erlauben, und
(b) Anheben des pH-Wertes auf eine zweite Stufe, die bei mindestens 10 liegt und hoch genug ist, aus der Reaktion der Zwischenverbindung mit Peroxid die Erzeugung eines Lichtblitzes zu bewirken, dessen Intensitat im Wesentlichen größer ist als die vor dem Anheben des pH-Wertes erzeugte.

2. Verfahren gemäß Anspruch 1, bei dem das Acridan Folgende Formel besitzt: 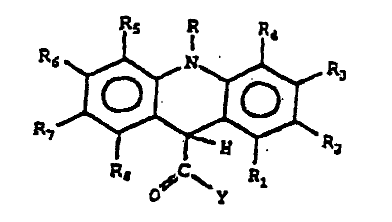 worin R aus Alkyl-, Heteroalkyl- und Aralkylgruppen ausgewählt ist, R₁ bis R₈ unabhangig voneinander aus Gruppen ausgewählt sind, die die Erzeugung von Licht ermoglichen, und Y eine austretende Gruppe ist.

3. Verfahren gemäß Anspruch 2, bei dem R₁ eine aus Alkyl-, Alkoxy- und Halogengruppen ausgewählte Gruppe ist.

4. Verfahren gemäß Anspruch 3, bei dem mindestens zwei Substituenten an dem Acridanring aus Alkyl- und Alkoxygruppen ausgewählt sind.

5. Verfahren gemäß Anspruch 1, bei dem das Acridan folgende Formel besitzt: 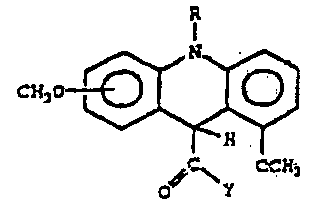 worin R aus Alkyl-, Heteroalkyl- und Aralkylgruppen ausgewählt ist und Y eine austretende Gruppe ist.

6. Verfähren gemäß Anspruch 2, bei dem die Gruppe Y eine mit mindestens einem Fluoratom substituierte Phenoxygruppe ist.

7. Verfahren gemäß Anspruch 1, bei dem das Acridan 2',3',6'-Trifluorphenyl-1,6-dimethoxy-10-methylacridan-9-carboxylat ist.

8. Verfahren gemäß Anspruch 1, bei dem der pH-Wert in Schritt (a) im Bereich zwischen 6 und 8,5 und der pH-Wert in Schritt (b) über 11 liegt.

9. Verfahren gemäß Anspruch 1 oder 2 zum Nachweis der Gegenwart oder Menge eines Analyts in einem Testverfahren, bei dem die Menge des erzeugten Lichts beobachtet und zu der Gegenwart oder Menge des Analyts in Beziehung gesetzt wird.

10. Verfahren gemäß Anspruch 9, bei dem der Analyt das Peroxid ist.

11. Verfahren gemäß Anspruch 9, bei dem der Analyt das Peroxidaseenzym ist.

12. Verfahren gemäß Anspruch 8, bei dem das Peroxidaseenzym an ein Mitglied eines aus der Gruppe bestehend aus Haptenen, Antigenen, Antikörpern und Oligonucleotiden ausgewählten speziellen Bindungspaares gebunden ist.

13. Kit zum Nachweis der Gegenwart oder Menge eines Analyts in einem Testverfahren durch eine Chemilumineszenzreaktion, bei der eine Zwischenverbindung entsteht, das Folgendes umfasst:
(a) eine Acridanverbindung,
(b) ein Peroxid in einer wässrigen Lösung eines pH-Wertes auf einer ersten Stufe im Bereich zwischen 5 und 9, die die Bildung einer Zwischenverbindung ermöglicht,
(c) ein Peroxidaseenzym entweder einzeln oder an eine analytbindende Verbindung gebunden, und
(d) ein Reagens zum Anheben des pH-Wertes der Lösung auf eine zweite Stufe, wobei das Licht in dem Testverfahren durch Umsetzen der Acridanverbindung mit dem Peroxid und dem Peroxidaseenzym zur Bildung einer Zwischenverbindung und anschließendes Anheben des pH-Wertes der Lösung mittels des Reagens auf die bei mindestens 10 liegende zweite Stufe nachgewiesen wird.

14. Kit gemäß Anspruch 13, bei dem das Acridan folgende Formel besitzt; 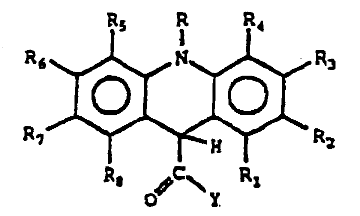 worin R aus Alkyl-, Heteroalkyl- und Aralkylgruppen ausgewählt ist, R₁ bis R₈ unablängig voneinander aus Gruppen ausgewählt sind, die die Erzeugung von Licht ermöglichen, und Y eine austretende Gruppe ist.

15. Kit gemäß Anspruch 14, bei dem sich das Acridan zusammen mit einer Phenolverbindung, die die Lichterzeugung aus dem Acridan verbessert, einer Peroxidverbindung, die an der Reaktion des Acridans mit der Peroxidase beteillgt ist, einem Chelatbildner, der verhindert, dass die Peroxidverbindung vor Zugabe der Peroxidase zu der Zusammensetzung reagiert, und einer oberflächenaktiven Substanz in einer Menge, die eine verbesserte Chemilumineszenz ermöglicht, in einer Reagenszusammensetzung befindet.

16. Kit gemäß Anspruch 14 oder 15, bei dem R₁ eine aus Alkyl-, Alkoxy- und Halogengruppen ausgewählte Gruppe ist.

17. Kit gemäß Anspruch 16, bei dem mindestens zwei Substituenten an dem Acridanring aus Alkyl- und Alkoxygruppen ausgewählt sind.

18. Kit gemäß Anspruch 14 oder 15, bei dem die Gruppe Y eine mit mindestens einem Fluoratom substituierte Phenoxygruppe ist.

19. Kit gemäß Anspruch 13, 14 oder 15, bei dem das Acridan folgende Formel besitzt: 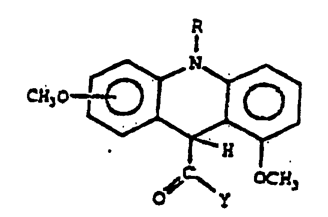 worin R aus Alkyl-, Heteroalkyl- und Aralkylgruppen ausgewählt ist und Y eine austretende Gruppe ist.

20. Kit gemäß einem der Ansprüche 13, 14 oder 15, bei dem das Acridan 2',3',6'-Trifluorphenyl-1,6-dimethoxy-10-methylacridan-9-carboxylat ist.

21. Verbindung, bei der es sich um 2',3',6'-Trifluorophenyl-1,6-dimethoxy-10-methylacridan-9-carboxylat handelt.

22. Verbindung, die 2',3',6'-Trifluorphenyl-1,6-dimenthoxy-10-methylacridin-9-carboxylat enthält.

## Revendications

1. Procédé de production d'une chimiluminescence comprenant les étapes consistant à :
(a) faire réagir un composé peroxyde et une enzyme peroxydase avec un acridane dans des conditions de temps, de température et de pH qui, à un premier niveau, est dans la plage de 5 à 9, et qui permettent la formation d'un composé intermédiaire ; et
(b) élever le pH jusqu'à un second niveau qui est d'au moins 10 et suffisamment élevé pour provoquer la production d'une bouffée de lumière provenant de la réaction du produit intermédiaire avec le peroxyde à une intensité substantiellement supérieure à celle produite avant élévation du pH.

2. Procédé selon la revendication 1, dans lequel l'acridane répond à la formule : 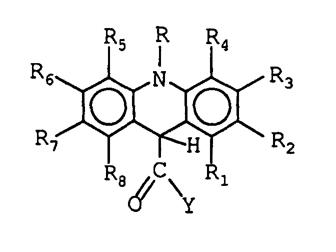 dans laquelle R est choisi parmi les groupes alkyle, hétéroalkyle et aralkyle, dans laquelle R₁ à R₈ sont choisis, indépendamment, parmi les groupes aptes à générer de la lumière et dans laquelle Y est un groupe labile.

3. Procédé selon la revendication 2, dans lequel R₁ est un groupe choisi parmi les groupes alkyle, alcoxy et halogène.

4. Procédé selon la revendication 3, dans lequel au moins deux substituants sur le cycle acridane sont choisis parmi les groupes alkyle et alcoxy.

5. Procédé selon la revendication 1, dans lequel l'acridane répond à la formule : 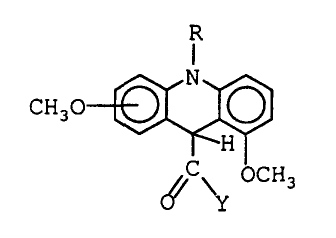 dans laquelle R est choisi parmi les groupes alkyle, hétéroalkyle et aralkyle et dans laquelle Y est un groupe labile.

6. Procédé selon la revendication 2, dans lequel le groupe Y est un groupe phénoxy substitué par au moins un atome de fluor.

7. Procédé selon la revendication 1, dans lequel l'acridane est un 1,6-diméthoxy-10-méthylacridane-9-carboxylate de 2',3',6'-trifluorophényle.

8. Procédé selon la revendication 1, dans lequel le pH dans l'étape (a) est dans la plage de 6 à 8,5 et le pH dans l'étape (b) est supérieur à 11.

9. Procédé selon la revendication 1 ou la revendication 2 qui sert à détecter la présence ou la quantité d'un analyte dans une procédure de dosage.

10. Procédé selon la revendication 9, dans lequel l'analyte est le peroxyde.

11. Procédé selon la revendication 9, dans lequel l'analyte est l'enzyme peroxydase.

12. Procédé selon la revendication 8, dans lequel l'enzyme peroxydase est liée à un élément d'une paire à liaison spécifique choisi dans le groupe composé des haptènes, des antigènes, des anticorps et des oligonucléotides.

13. Trousse pour détecter la présence ou la quantité d'un analyte dans une procédure de dosage par une réaction chimioluminescente qui produit un composé intermédiaire, comprenant :
(a) un composé acridane ;
(b) un peroxyde dans une solution aqueuse à un pH qui, à un premier niveau, est dans la plage de 5 à 9 et qui permet la formation d'un composé intermédiaire ;
(c) une enzyme peroxydase soit seule, soit liée à un composé se liant à un analyte ; et
(d) un réactif pour élever le pH de la solution jusqu'à un second niveau,
la lumière étant détectée dans la procédure de dosage en faisant réagir le composé acridane avec le peroxyde et l'enzyme peroxydase de manière à former un composé intermédiaire et en élevant, par la suite, le pH de la solution jusqu'au second niveau qui est d'au moins 10, avec le réactif.

14. Trousse selon la revendication 13, dans laquelle l'acridane répond à la formule : 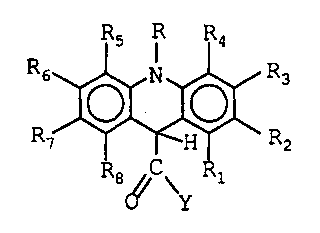 dans laquelle R est choisi parmi les groupes alkyle, hétéroalkyle et aralkyle, dans laquelle R₁ à R₈ sont choisis, indépendamment, parmi les groupes aptes à générer de la lumière et dans laquelle Y est un groupe labile.

15. Trousse selon la revendication 14, dans laquelle l'acridane est contenu dans une composition de réactif avec un composé phénolique qui renforce la production de lumière générée par l'acridane ; un composé peroxyde qui participe à la réaction de l'acridane avec la peroxydase ; un agent de chélation qui empêche le composé peroxyde de réagir avant l'addition de la peroxydase à la composition ; et un tensioactif en une quantité apte à fournir une chimiluminescence améliorée.

16. Trousse selon les revendications 14 ou 15, dans laquelle R₁ est un groupe choisi parmi les groupes alkyle, alcoxy et halogène.

17. Trousse selon la revendication 16, dans laquelle au moins deux substituants sur le cycle acridane sont choisis parmi les groupes alkyle et alcoxy.

18. Trousse selon la revendication 14 ou 15, dans laquelle le groupe Y est un groupe phénoxy substitué par au moins un atome de fluor.

19. Trousse selon l'une quelconque des revendications 13, 14 ou 15, dans laquelle l'acridane répond à la formule : 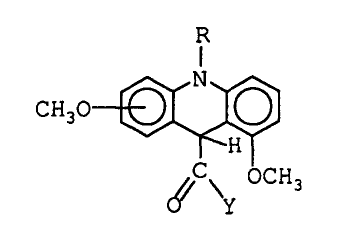 dans laquelle R est choisi parmi les groupes alkyle, hétéroalkyle et aralkyle et dans laquelle Y est un groupe labile.

20. Trousse selon l'une quelconque des revendications 13, 14 ou 15, dans laquelle l'acridane répond à la formule du 1,6-diméthoxy-10-méthylacridane-9-carboxylate de 2',3',6'-trifluorophényle.

21. Composé qui est le 1,6-diméthoxy-10-méthylacridane-9-carboxylate de 2',3',6'-trifluorophényle.

22. Composé qui contient un 1,6-diméthoxy-10-méthylacridinium-9-carboxylate de 2',3',6'-trifluorophényle.
